# EUROPEAN PATENT APPLICATION

(11) **EP 2 436 766 A1**
(43) Date of publication of application: **04.04.2012**
(21) Application number: 10182518.0
(22) Date of filing: 29.09.2010
(51) Int. Cl.: C12N 15/10, C40B 40/02, C40B 40/08, C40B 50/06

(54) **Means and methods for improved protein interaction screening**

(71) Applicant: Deutsches Krebsforschungszentrum, 69120 Heidelberg (DE)
(72) Inventor: Kögl, Manfred, 2340 Mödling (AT); Asser-Kaiser, Manfred, 69123 Heidelberg (DE)
(74) Representative: Dick, Alexander

(57) **Abstract**

The current invention is concerned with tools for protein interaction screening. Specifically, it relates to a polynucleotide comprising a barcode sequence flanked by two primer binding sequences and further comprising a sequence encoding a protein of interest, a vector comprising said polynucleotide, and a host cell comprising said polynucleotide or vector. It further pertains to a DNA obtainable by cross-over PCR from a two polynucleotides of the invention and with a method for identifying two proteins of interest that interact, comprising a) providing at least one first protein of interest expressed from a first polynucleotide of the invention and at least one second protein of interest expressed from a second polynucleotide of the invention, b) incubating said first protein of interest and second protein of interest under conditions allowing the formation of protein-protein interactions, c) determining binding between the first and the second protein of interest, and d) sequencing the barcode sequences of the first and the second polynucleotide.

## Description

The current invention is concerned with tools for protein interaction screening. Specifically, it relates to a polynucleotide comprising a barcode sequence flanked by two primer binding sequences and further comprising a sequence encoding a protein of interest, a vector comprising said polynucleotide, and a host cell comprising said polynucleotide or vector. It further pertains to a DNA obtainable by cross-over PCR from two polynucleotides of the invention and with a method for identifying two proteins of interest that interact, comprising a) providing at least one first protein of interest expressed from a first polynucleotide of the invention and at least one second protein of interest expressed from a second polynucleotide of the invention, b) incubating said first protein of interest and second protein of interest under conditions allowing the formation of protein-protein interactions, c) determining binding between the first and the second protein of interest, and d) sequencing the barcode sequences of the first and the second polynucleotide.

Identifying protein-protein interactions is an important task in biological and medical sciences. Interactions of proteins provide important clues about cellular signaling networks and metabolic and genetic regulation mechanisms. Moreover, inhibitors of protein-protein interactions are important pharmaceuticals, so there is a direct impact of the elucidation of protein-protein interactions on medicine.

Screening methods can be separated essentially into two groups: in the first group, interacting proteins are identified as such, i.e. the identification method comprises a step wherein the information comprised in the protein molecule itself is used for identification. Examples for methods falling into this group are immuneprecipitation, surface plasmon resonance screening, or peptide-array screening methods. Identification of proteins is accomplished e.g. by sequence determination through Edman-degradation or mass-spectrometry of peptides derived from the protein.

In the second group, the protein and the sequence coding for it are coupled tightly, e.g. by maintaining both in the same cell. In these assays, exemplified by phage display (Bratkovic T. (2010) "Progress in phage display: evolution of the technique and its application" Cell Mol Life Sci. Mar;67(5):749-767) or the various flavours of two-hybrid screening methods (Dove and Hochschild (2004), "A bacterial two-hybrid system based on transcription activation", Methods Mol Biol. 261:231-4; Brückner et al. (2009), "Yeast two-hybrid, a powerful tool for systems biology", Int J Mol Sci. 10(6):2763-8; Lievens et al. (2009) "Mammalian two-hybrids come of age", Trends Biochem Sci. 34(11):579-88; Lalonde et al. (2008), "Molecular and cellular approaches for the detection of protein-protein interactions: latest techniques and current limitations", Plant J. 53(4):610-35), bacteriophages or cells containing interacting proteins are identified and the DNAs encoding said proteins are extracted. Identification of proteins is accomplished by DNA sequencing.

To date, screening assays of the second group are usually performed keeping one interaction partner constant (the "bait" protein), whereas the other interaction partner normally is provided as a library of proteins (the "prey" proteins). Prey proteins interacting with the bait are identified by sequencing the nucleic acid sequences encoding the said prey proteins. Efficieny of such assays is limited since single clones have to be isolated to make sequencing possible. So, it is desirable to develop protein-protein interaction screening methods allowing for a more efficient screening.

The technical problem underlying the present invention can thus be seen as the provision of means and methods complying with the aforementioned needs. The technical problem is solved by the embodiments characterized in the claims and hereinafter.

Accordingly, the current invention relates to a polynucleotide comprising a barcode sequence flanked at the 5' side by a first and at the 3' side by a second primer binding sequence and a nucleic acid sequence encoding a protein of interest.

As used herein, the term "polynucleotide" relates to a polymer comprising at least 50, at least 100, or at least 250 nucleotides comprising the nucleic acid sequences as described herein below. Preferably, the polynucleotide is DNA or RNA. The polynucleotides of the present invention either essentially consist of the nucleic acid sequences described below or comprise said nucleic acid sequences. Thus, they may contain further nucleic acid sequences as well.

The polynucleotide of the present invention shall be provided, preferably, either as an isolated polynucleotide (i.e. isolated from its natural context) or in genetically modified form or comprised in a host cell of the current invention. The polynucleotide, preferably, is DNA including cDNA, or RNA. The term encompasses single as well as double stranded polynucleotides in linear or circular form. Moreover, comprised are also chemically modified polynucleotides including naturally occurring modified polynucleotides such as glycosylated or methylated polynucleotides or artificial modified ones such as biotinylated polynucleotides.

The term "barcode sequence" relates to a nucleic acid sequence comprised by the polynucleotide of the current invention allowing for unambiguous identification of the said polynucleotide and, thus, also allowing for identification of the other sequences comprised by the polynucleotide having said barcode sequence. Preferably, a barcode sequence consists of a sequence of at least ten, at least eleven, at least twelve, at least thirteen, at least fourteen, at least fifteen, at least sixteen, at least eighteen, at least twenty consecutive randomly assembled nucleotides. Preferably, said barcode sequence is theoretically unique. It is well known in the art how random sequences can be achieved in oligonucleotide synthesis. It is to be understood that the number of different polynucleotide molecules theoretically possible is directly dependent on the length of the barcode sequence; e.g., if a DNA barcode with randomly assembled Adenine, Thymidine, Guanosine and Cytidine nucleotides is used, the theoretical maximal number of barcode sequences possible is 1,048,576 for a length of ten nucleotides, and is 1,073,741,824 for a length of fifteen nucleotides. Preferably, the length of the barcode sequences is selected such that the number of unique sequences theoretically possible is at least as high as the number of preys used in a pool of sequences. The person skilled in the art knows how to adopt the length of the barcode sequence. Preferably, said barcode sequences are inserted into a pre-defined nucleotide sequence, e. g. a restriction enzyme recognition site, such that the start point of said barcode sequence is pre-determined unambiguously.

A "primer binding sequence" as used herein relates to a nucleic acid sequence known to specifically hybridize to a predefined PCR primer under conditions typically used in PCR or other polynucleic acid amplifying methods. Thus, the primer binding sequence of the current invention consists of at least fifteen, at least sixteen, at least seventeen, at least eighteen consecutive nucleotides with a known sequence. Preferably, the polynucleotide of the invention comprises two primer binding sequences, wherein the melting temperature of the primer binding sequences differs by no more than six, no more than five, no more than four, no more than tree, or no more than two degrees Celsius. More preferably, the first and the second primer binding sequence differ in their nucleotide sequence to such an extent that an oligonucleotide specifically hybridizing to the first primer binding sequence does not hybridize specifically to the second primer binding sequence and that a primer specifically hybridizing to the second primer binding sequence does not hybridize specifically to the first primer binding sequence. Most preferably, the nucleotide sequences of the primer binding sequences are selected from the list consisting of M13 primer binding sequence (SEQ ID NO: 1), SK primer binding sequence (SEQ ID NO: 2), the primer A for 454-sequencing (SEQ ID NO:3), the primer B for 454-sequencing (SEQ ID NO: 4)

As used herein, the term "flanked" means being arranged in close proximity. Preferably, the barcode sequence of the current invention is flanked by a first and a second primer binding sequence such that a nucleic acid produced by PCR using primers hybridizing specifically to said first and second primer binding sequences will consist of no more than 300, no more than 250, no more than 200, no more than 150, no more than 100, no more than 75, or no more than 50 nucleotides. More preferably, the first and the second primer binding sequence are separated from the barcode sequence by no more than ten, eight, six, five, four, three, or two nucleotides.

The terms "5' side" and "3' side" of a polynucleotide sequence are well known to the skilled artisan. It is to be understood that the isolated barcode sequence of the current invention per se has no orientation with respect to a 5' or 3' side, such that, with respect to the placement of the first and the second primer binding sequence relative to the barcode sequence, the terms 5' side and 3' side are a matter of convention. In each case, however, the first and the second primer binding sequence are placed on different sides relative to the barcode sequence.

The "protein of interest" of the current invention is a protein or a part thereof suspected to interact with another protein or part thereof. Preferably, the protein of interest encoded on the polynucleotide of the current invention is comprised in a multitude of polynucleotides, wherein the polynucleotides comprise unique combinations of barcode sequence and protein of interest (a pool). More preferably, the unique combination of barcode sequence and protein of interest encoded on the same polynucleotide has been determined for the member polynucleotides of said pool, e.g. by sequencing. It is to be understood that each of the unique combinations can be present in the pool several times, e.g. after amplification of said combinations in the cloning process. Preferably, each protein encoded by the genome of an organism is prima facie suspected to interact with another protein of said organism; e.g. for baker's yeast it has been estimated that on average a protein will interact with approximately 1.5 (Ito,T. et al., (2001) "A comprehensive two-hybrid analysis to explore the yeast protein interactome". Proc. Natl Acad. Sci. USA, 98, 4569-4574) to five (Grigoriev, A. (2003) "On the number of protein-protein interactions in the yeast proteome". Nucl Acid Res, 31 (14), 4157-4161) other proteins. Thus, preferably, a library of cDNA from an organism, an EST (expressend sequence tags) library or a genomic library are used as a source of polynucleotides coding for proteins of interest. More preferably, a multitude of member sequences of one of said libraries are used as sequences coding for proteins of interest.

Preferably, the proteins of interest are exspressed as fusion proteins. Such fusion proteins in a preferred embodiment of the polynucleotide of the invention encoding them comprise as additional part a transcriptional activation or a DNA-binding domain or an RNA-binding domain. Moreover, further comprised may be nucleic acid sequences encoding polypeptides for monitoring expression (e.g., green, yellow, blue or red fluorescent proteins, alkaline phosphatase and the like) or so called "tags" which may serve as a detectable marker or as an auxiliary measure for purification purposes. Tags for the different purposes are well known in the art and comprise FLAG-tags, 6-histidine-tags, MYC-tags and the like.

The polynucleotide of the current invention, preferably, is used in a method for the identification of protein-protein-interactions (identification methods), i.e. a method wherein proteins are incubated under conditions allowing said proteins to interact and wherein suitable means and methods for the identification of interacting proteins are provided. The polynucleotide of the invention, thus, encodes a protein of interest in an expressible form, i.e. in a form that allows for a protein to be synthesized from said polynucleotide under appropriate conditions known to the skilled person. It is to be understood that the polynucleotide of the current invention is used specifically in identification methods comprising an identification step wherein the information on the identity of the interaction partner is derived from the polynucleotide encoding said interaction partner. Examples of identification methods comprising said identification step are the methods referred to as phage display and two-hybrid screening. The methods of phage display and the suitable variations of two-hybrid-screening, like e.g. yeast two-hybrid screening, E. coli two-hybrid screening, mammalian two-hybrid screening, and split-ubiquitin two-hybrid screening are known to the skilled artisan. Most preferably, the method is yeast two-hybrid screening.

As used herein, the term "transcriptional activation domain" relates to a protein or to a peptide derived thereof activating transcription when brought in close proximity to the promoter of a gene; preferably, said activation is mediated by binding of the transcriptional activation domain to RNA polymerase. Preferably, the transcriptional activation domain is the activation domain of the Gal4 protein of baker's yeast or the herpes simplex virus protein VP16 transcriptional activation domain.

The term "fusing" a first polypeptide to a second polypeptide as used herein relates to covalently joining a first nucleotide sequence coding for a first polypeptide to a second nucleotide sequence coding for a second polypeptide in such a way that the two polypeptides are expressed as one contiguous polypeptide in the host cell.

The "DNA binding domain" of the current invention is a protein or a peptide derived therefrom specifically binding to a known specific DNA sequence. Preferably, the DNA binding domain is the DNA binding domain of the Gal4 protein of baker's yeast, or the DNA binding domain of the Escherichia coli lexA protein, or the DNA binding domain of the human NFkB transcription factor.

The current invention furthermore relates to a vector comprising the polynucleotide of the current invention.

The term "vector", preferably, encompasses phage, plasmid, viral or retroviral vectors as well as artificial chromosomes, such as bacterial or yeast artificial chromosomes. The vector encompassing the polynucleotides of the present invention, preferably, further comprises at least one selectable marker for propagation and/or selection in a host. The vector may be incorporated into a host cell by various techniques well known in the art. For example, a plasmid vector can be introduced in a precipitate such as a calcium phosphate precipitate or rubidium chloride precipitate, or in a complex with a charged lipid or in carbon-based clusters, such as fullerens. Alternatively, a plasmid vector may be introduced by heat shock or electroporation techniques. Should the vector be a virus, it may be packaged in vitro using an appropriate packaging cell line prior to application to host cells. Retroviral vectors may be replication competent or replication defective. In the latter case, viral propagation generally will occur only in complementing host/cells.

More preferably, in the vector of the invention the polynucleotide of the invention is operatively linked to expression control sequences allowing expression of the protein of interest in prokaryotic or eukaryotic cells or isolated fractions thereof. Expression of said polynucleotide comprises transcription of the polynucleotide, preferably into a translatable mRNA. Regulatory elements ensuring expression preferably in eukaryotic cells, more preferably in yeast or mammalian cells, are well known in the art. They, preferably, comprise regulatory sequences ensuring initiation of transcription and, optionally, poly-A signals ensuring termination of transcription and stabilization of the transcript. Additional regulatory elements may include transcriptional as well as translational enhancers. Possible regulatory elements permitting expression in prokaryotic host cells comprise, e.g., the *lac, trp* or *tac* promoter in *E. coli,* and examples for regulatory elements permitting expression in eukaryotic host cells are the *ADH* or *GAL1* promoter in yeast or the CMV-, SV40-, RSV-promoter (Rous sarcoma virus), CMV-enhancer, SV40-enhancer or a globin intron in mammalian and other animal cells. Moreover, inducible expression control sequences may be used in an expression vector encompassed by the present invention. Such inducible vectors may comprise *tet* or *lac* operator sequences or sequences inducible by heat shock or other environmental factors. Suitable expression control sequences are well known in the art. Beside elements which are responsible for the initiation of transcription such regulatory elements may also comprise transcription termination signals, such as the SV40-poly-A site or the tk-poly-A site, downstream of the polynucleotide. In this context, suitable expression vectors are known in the art such as Okayama-Berg cDNA expression vector pcDV1 (Pharmacia), pBluescript (Stratagene), pCDM8, pRc/CMV, pcDNA1, pcDNA3 (Invitrogene) or pSPORT1 (GIBCO BRL), pGBT9, pGAD424, pGBKT7, pGADT7, pAS2, pACT2. Methods which are well known to those skilled in the art can be used to construct recombinant vectors; see, for example, the techniques described in Sambrook, Molecular Cloning A Laboratory Manual, Cold Spring Harbor Laboratory (1989) N.Y. and Ausubel, Current Protocols in Molecular Biology, Green Publishing Associates and Wiley Interscience, N.Y. (1994).

As used herein, the term "selectable marker" relates to a gene conferring a selective advantage to the cell expressing said gene under certain conditions, i.e. selective conditions. Preferably, only cells expressing the selectable marker can grow under the selective conditions. Non-limiting examples of selectable markers and the respective selective conditions are: the ampicillin resistance gene in E. coli and the presence of ampicillin in the growth medium; the kanamycin resistance gene in E. coli and the presence of kanamycin in the growth medium; the neomycin resistance gene in a mammalian cell and the presence of neomycin in the medium, the TRP1 auxotrophic marker for yeast cells bearing a mutation in the chromosomal TRP1 gene, and the LEU2 auxotrophic marker for yeast cells bearing a mutation in the chromosomal LEU2 gene.The skilled artisan knows other combinations of selectable markers and suitable conditions for selection. Especially, the skilled person knows how the specific conditions, like, e.g. the concentration of compounds, can be adjusted.

The invention also relates to a host cell comprising the polynucleotide or the vector of the current invention.

The term "host cell", preferably, relates to a cell maintained in vitro in a suitable cultivation medium. Maintaining as used in this context relates to incubating a target cell under conditions suited for the growth of the respective target cell, which vary with the type of host cell and which are well known in the art. Notwithstanding the above, the host cell of the current invention may be maintained in vitro under conditions suited for selecting for the expression of a selectable marker in the cell, said conditions being adverse for host cells not expressing said selectable marker. Preferably, the host cell is a bacterial or eukaryotic cell. More preferably, the host cell is a mammalian or fungal cell. Most preferably, the host cell is a cell from Saccharomyces cerevisiae (baker's yeast).

The host cell of the current invention preferably comprises a genetic defect making said cell unable to grow in the absence of a certain nutrient. More preferably, said nutrient is an amino acid or nucleotide, most preferably adenine, tryptophane, uracil histidine or leucine. Preferably, the host cell further comprises a gene complementing said genetic defect under the control of an expression control sequence, said expression control sequence activating transcription, and thus complementation of the genetic defect, specifically if two interacting proteins are present in the host cell.

Preferably, the host cell further comprises a polynucleotide of the current invention comprising a fusion of a protein of interest to a transcriptional activation domain ("AD-fusion"); or the host cell further comprises a polynucleotide of the current invention comprising a fusion of a protein of interest to a DNA binding domain ("BD-fusion"). More preferably, the host cell comprises an AD-fusion and a BD-fusion in the same cell. Most preferably, said host cell comprising an AD-fusion and a BD-fusion is generated by fusing a host cell comprising an AD-fusion to a host cell of the opposite mating type comprising a BD-fusion.

The current invention further relates to a DNA obtainable by cross-over PCR from a first polynucleotide of the current invention and a second polynucleotide of the current invention.

As used herein, the term "cross-over PCR" relates to a variant of overlap-extension PCR (Higuchi et al. (1988), "A general method of in vitro preparation and specific mutagenesis of DNA fragments: study of protein and DNA interactions", Nucleic Acids Res 16 (15), 7351 - 7367, Example 1) wherein the two partial products cross-priming to generate the full-length product are produced from two different polynucleotides of the current invention. It is preferable for an effective cross-over PCR that the two polynucleotides according to the invention comprise one primer binding sequence identical for both polynucleotides or at least one primer binding sequence on each polynucleotide which is capable of hybridizing under PCR conditions with the other one, respectively, and one primer binding sequence specific for each polynucleotide (as e.g. in example 1). The general structure of the DNA anticipated to be produced from said PCR, preferably, is: primer binding sequence (specific for the first polynucleotide) - barcode of the first polynucleotide - primer binding sequence (identical for both polynucleotides) - barcode of the second polynucleotide - primer binding sequence (specific for the second polynucleotide). It is to be understood that not all DNA molecules produced in cross-over PCR will be of this structure. A significant portion of the DNA products of cross- over PCR may be side products not corresponding to the aforesaid general structure. However, the amount of products of the structure as described above shall be high enough to allow the sequence of said anticipated product to be determined by one of the methods well known to the skilled artisan. As exemplified in example 1, yield and specificity of the amplification can be increased by performing a second PCR amplification on the products of the cross-over PCR using primers that hybridize within the anticipated product.

According to the invention, precautions are taken to keep first and second polynucleotides in close spatial proximity in those cases where interaction of the proteins encoded on said polynucleotides occurred. For example, in the case of two-hybrid interaction studies, each yeast cell will comprise two different polynucleotides, one of said polynucleotides coding for an AD-fusion protein and the other one coding for a BD-fusion protein. Said two polynucleotides are in close proximity, yet separable from other polynucleotides of the invention, e.g. by growing each cell comprising polynucleotides coding for interacting proteins into clones on solid growth media or in separate wells of microtiter plates or by suspending single cells into droplets as it is the case with the Roche 454 emulsion PCR technology. At the same time, polynucleotides from one cell are prevented from mixing with different polynucleotides comprised in a different cell. Thus, if cross-over PCR is applied to the two polynucleotides comprised in a single cell or in a clone grown from said cell, the barcode sequences from both of said two polynucleotides are comprised in the DNA produced in said cross-over PCR.

Preferably, the protein of interest encoded on the first polynucleotide is different from the protein of interest encoded on the second polynucleotide, thus leading to the identification of an interaction between two different proteins. Preferably, the first protein of interest is fused to a transcriptional activation domain and the second protein of interest is fused to a DNA binding domain, so that the interaction between the first protein of interest and the second protein of interest leads to the epression of a selectable marker in a two-hybrid screening system.

The current invention encompasses a method for identifying two proteins of interest that interact, comprising a) providing at least one first protein of interest expressed from a first polynucleotide of the invention and at least one second protein of interest expressed from a second polynucleotide of the invention, b) incubating said first protein of interest and second protein of interest under conditions allowing the formation of protein-protein interactions, c) determining binding between the first and the second protein of interest, and d) determining the barcode sequences of the first and the second polynucleotide, whereby two proteins of interest that interact are identified.

The term "incubating" as used in the context of the methods of the present invention is understood by the skilled person. Preferably, the term relates to bringing proteins of interest into close proximity and to keeping them under conditions which are generally suitable for protein-protein interactions. It is to be understood by the skilled person that not all protein-protein interactions can be detected under a certain set of conditions. More preferably, incubating proteins of interest relates to expressing said proteins in a living cell so that interaction can occur in a compartment of the cell. Most preferably, said compartment is the compartment comprising the genetic information of the cell, e.g. the cytoplasm of a bacterial cell or the nucleus of a cell from baker's yeast.

"Determining binding" as used herein, relates to detecting physicochemical interaction between two proteins of interest. The skilled person knows how the interaction between two proteins can be determined. As detailed supra, determining binding is accomplished by identification methods comprising an identification step wherein the information on the identity of the interaction partner is derived from the polynucleotide encoding said interaction partner. Examples for such identification methods are: Phage display, where the binding is determined by the adhesion of a bacteriophage to a bacterial cell and the information on the protein mediating said interaction is encoded on the genome of the bacteriophage; Two-hybrid-screening, where interaction is determined by activation of transcription of a reporter gene and the information on the interacting proteins is encoded on expression vectors comprised in the host cell.

In two-hybrid-screening, a preferred reporter gene is a gene complementing an auxotrophic marker, e.g. a wild-type allele of a gene involved in the biosynthesis of an essential amino acid or an essential nucleotide. More preferably, said reporter gene is the HIS3 gene of Saccharomyces cerevisiae, coding for imidazoleglycerol-phosphate dehydratase, or the URA3 gene of Saccharomyces cerevisiae, coding for orotidine 5-phosphate decarboxylase, or the ADE2 gene coding for phosphoribosylaminoimidazole carboxylase activity. It is to be understood that the host cell comprises no or only non-functional copies of said gene or genes in such case, such that only cells comprising interacting proteins and, thus, activating expression of said wild-type allele, are able to proliferate in the absence of said amino acid or nucleotide. Other preferred reporters include enzymes which have an activity which can be detected by convenient methods, such as the MEL1 gene coding alpha-galactosidase whose activity can be detected by the use substrates which have an alpha-galactosidic bond and which upon cleavage of said bond display a change in color or fluorescence, such as 4-methylumbelliferyl-alpha-D-galactoside, or the bacterial lacZ gene coding for beta-galactosidase,

Preferably, in yeast two-hybrid-screening both polynucleotides of the method are transferred into the host cell by one of the transformation methods known in the art. More preferably, first two libraries of host cells are generated, wherein the first pool of host cells comprises a pool of polynucleotides coding for proteins of interest fused to an activation domain and wherein the host cells are of one mating type, and wherein the second pool of host cells comprises a pool of polynucleotides coding for proteins of interest fused to a DNA binding domain and wherein the host cells are of the opposite mating type relative to the host cells of the first pool. Thus, after statistically mating host cells from the first pool to host cells from the second pool a multitude of host cells is obtained, each comprising a statistical combination of first polynucleotide and second polynucleotide.

Determining barcode sequences is readily understood by the skilled person. Preferably, the term relates to determining the nucleotide sequences of the barcode sequence of the first and the second polynucleotide which were found to code for interacting proteins of interest. Preferably, said barcode sequences of said first and second polynucleotide are sequenced using the polynucleotides or their amplification products as templates. More preferably, the said two barcode sequences are combined into the DNA of the current invention by cross-over PCR and said DNA is used as a template for sequencing as specified elsewhere herein.

All references cited throughout this specification are herewith incorporated by reference with respect to their specific disclosure content referred to in this specification..

The figures show:

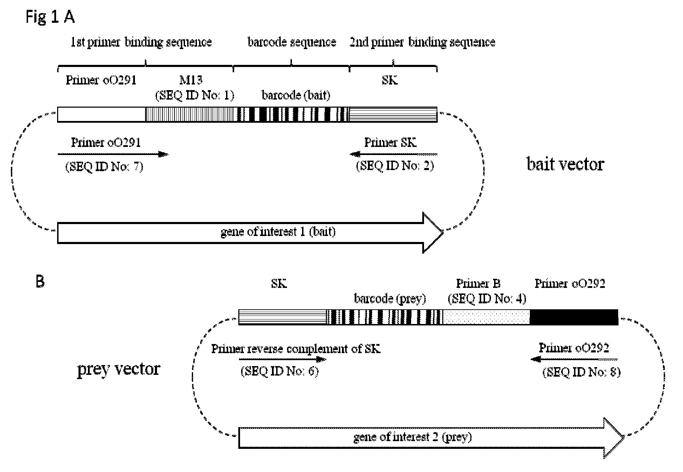

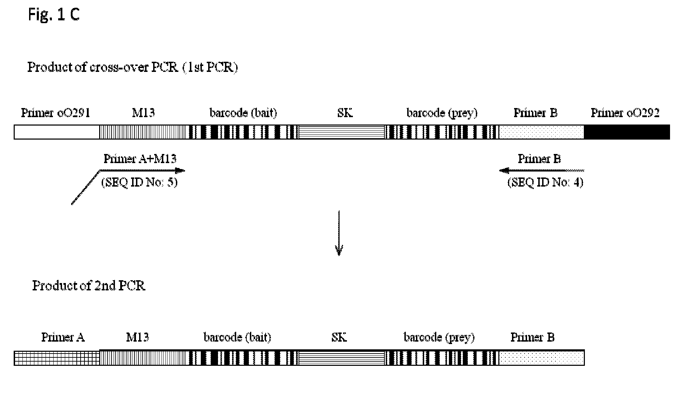

Figure 1: A and B, schematic representation of the bait and prey vector. Only relevant parts of the vectors are shown. The priming positions of the oligos used in Example 1 are indicated. A, sequences surrounding the barcode sequence of the bait vector (SEQ ID NO:9), B, sequences surrounding the barcode sequence of the prey vector (SEQ ID NO:10). C, schematic representation of anticipated products of the first and second PCR reactions. Primer A and Primer B are primers generally used in 454 sequencing (Roche).

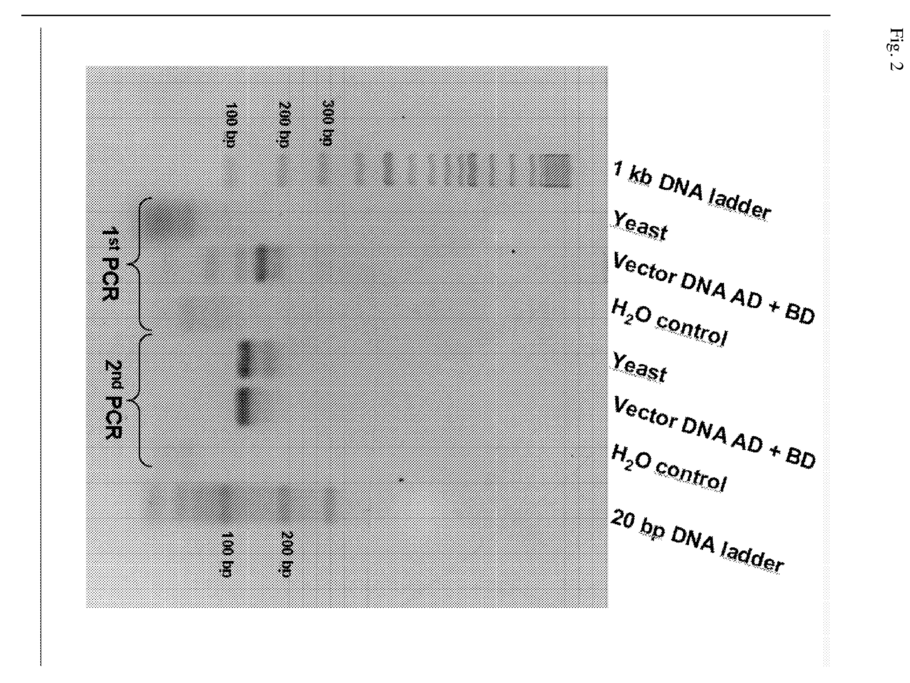

Figure 2: The products of a cross-over PCR are shown after size separation by agarose gel electrophoresis.

The following examples shall illustrate the invention. However, they shall not be construed, whatsoever, as limiting the scope.

### Examples

### Example 1: Cross-over PCR

A PCR reaction is done in which the bait and the prey plasmids are used as templates. The primers used in the example are indicated in Figure 1. The reaction is performed in two steps: In the first PCR reaction, the two plasmids are used and the following primers: SEQ ID NO: 2, SEQ ID NO: 7, SEQ ID NO: 6 and SEQ ID NO: 8. This will result in the amplification of the cross-over PCR product. A smaller fragment of this product can then be re-amplified in a second PCR reaction with two nested primers (SEQ ID NO: 4 and SEQ ID NO: 5). The second PCR reaction helps in generating sufficient amounts of the PCR product for further analysis by next generation sequencing.

The products of the two PCR Reactions is shown in Figure 2. In the experiment shown, the fusion PCR was done with the purified bait and prey vectors as templates, as well as with yeast cells as templates, as indicated.

The PCR reactions are done as follows:
1^{st} PCR:

| | |
|---|---|
| 10 x NH₄ reaction buffer: | 5 µl |
| 100 mM dNTPs | 0.5 µl |
| 50 mM M_{g}Cl₂ | 2µl |
| Primer: SEQ ID NO: 2 (10 mM) | 2µl |
| Primer: SEQ ID NO: 7 (10 mM) | 2µl |
| Primer: SEQ ID NO: 6 (10 mM) | 2µl |
| Primer: SEQ ID NO: 8 (10 mM) | 2µl |
| BIOTAQ Red Polymerase (1 µ/µ1) | 1 µl |
| Yeast cells: | 4×10⁶ cells |
| or Vector DNA (SEQ ID NO: 11 and 12) ad 50 µl with ddH₂O | ∼ 150 ng of each vector |

2^{nd} PCR:

| | |
|---|---|
| 10 x NH₄ reaction buffer: | 5 µl |
| 100 mM dNTPs | 0.5 µl |
| 50 mM MgCl₂ | 2µl |
| Primer: SEQ ID NO: 4 (10 mM) | 2µl |
| Primer: SEQ ID NO: 5 (10 mM) | 2µl |
| BIOTAQ Red Polymerase (1 u/µl): | 1 µl |
| Template: PCR product of 1^{st} PCR: | 1 µl |
| ddH₂O: | 36.5 µl |

PCR programs:
1^{st} PCR:

| | | |
|---|---|---|
| 1) initial denaturation: | 95°C | 5 min |
| 2) denaturation: | 95°C | 30 sec |
| 3) annealing: | 65°C | 30 sec |
| 4) elongation: | 72°C | 30 sec |
| 2) to 4) repeated for 35 cycles | | |
| 5) final elongation: | 72°C | 7 min |

2^{nd} PCR:

| | | |
|---|---|---|
| 1) initial denaturation: | 95°C | 5 min |
| 2) denaturation: | 95°C | 30 sec |
| 3) annealing: | 65°C | 30 sec |
| 4) elongation: | 72°C | 30 sec |
| 2) to 4) repeated for 30 cycles | | |
| final elongation: | 72°C | 7 min |

### Example 2:

Vectors pGBT9-GW-BC (SEQ ID NO: 9) and pGAD424-GW-BC (SEQ ID NO: 10). These vectors contain cloning sites which allow the insertion of barcode sequences. Barcode sequences are inserted between bases 2619 and 2620 in pGBT9-GW-BC and between bases 2576 and 2577 in pGAD424-GW-BC.

## Claims

1. A polynucleotide comprising a barcode sequence flanked at the 5' side by a first and at the 3' side by a second primer binding sequence and a nucleic acid sequence encoding a protein of interest.

2. The polynucleotide of claim 1, wherein the first and second primer binding sequence comprise at least 15 nucleotides of pre-determined sequence in close proximity to the barcode sequence.

3. The polynucleotide of claim 1 or 2, wherein the polynucleotide can be used in a method selected from the group consisting of: phage display and two-hybrid screening.

4. The polynucleotide of any one of claims 1 to 3, wherein the protein of interest is fused to a transcriptional activation domain.

5. The polynucleotide of any one of claims 1 to 3, wherein the protein of interest is fused to a DNA binding domain.

6. A vector comprising the polynucleotide of any one of claims 1 to 5.

7. The vector of claim 6, wherein said vector is further comprising a selectable marker.

8. A host cell comprising the polynucleotide of any one of claims 1 to 5 or the vector of claim 6 or 7.

9. The host cell of claim 8, Wherein said host cell is comprising the polynucleotide of claim 4 and the polynucleotide of claim 5.

10. A DNA obtainable by cross-over PCR from a first polynucleotide according to claim 1 and a second polynucleotide according to claim 1.

11. The DNA of claim 10, wherein the protein of interest encoded on the first polynucleotide differs from the protein of interest encoded on the second polynucleotide.

12. The DNA of claim 10 or 11, wherein the first polynucleotide is a polynucleotide of claim 4 and the second polynucleotide is a polynucleotide of claim 5.

13. A method for identifying two proteins of interest that interact, comprising
a) providing at least one first protein of interest expressed from a first polynucleotide of any one of claims 1 to 5 and at least one second protein of interest expressed from a second polynucleotide of any one of claims 1 to 5,
b) incubating said first protein of interest and second protein of interest under conditions allowing the formation of protein-protein interactions,
c) determining binding between the first and the second protein of interest, and
d) determining the barcode sequences of the first and the second polynucleotide, whereby two proteins of interest that interact are identified.

14. The method of claim 13, wherein the first polynucleotide is a polynucleotide of claim 4 and the second polynucleotide is a polynucleotide of claim 5.

15. The method of claim 13 or 14, wherein step c) further comprises enriching of at least one combination of a first protein of interest and a second protein of interest, achieved by selecting for expression activation of at least one gene complementing at least one auxotrophic marker.

16. The method of any one of claims 13 to 15, wherein step a) further comprises mating of a first yeast strain comprising a first polynucleotide of any one of claims 1 to 4 to a second yeast strain comprising a second polynucleotide of any one of claims 1 to 3 or 5.

17. The method of any one of claims 11 to 14, wherein step c) comprises the synthesis of a DNA according to claim 10 by cross-over PCR.


SEQUENCE LISTING

<110> Deutsches Krebsforschungszentrum

<120> Means and methods for improved protein interaction screening

<130> DK10054EP

<160> 10

<170> PatentIn version 3.3

<210> 1
<211> 17
<212> DNA
<213> Artificial

<220>
<223> M13 primer binding sequence

<400> 1
tgtaaaacga cggccag 17

<210> 2
<211> 20
<212> DNA
<213> Artificial

<220>
<223> SK primer binding sequence

<400> 2
gatccactag ttctagagcg 20

<210> 3
<211> 25
<212> DNA
<213> Artificial

<220>
<223> primer A for 454-sequencing

<400> 3
cgtatcgcct ccctcgcgcc atcag 25

<210> 4
<211> 25
<212> DNA
<213> Artificial

<220>
<223> primer B for 454-sequencing)

<400> 4
ctatgcgcct tgccagcccg ctcag 25

<210> 5
<211> 43
<212> DNA
<213> Artificial

<220>
<223> Primer with Primer A and M13 Sequence

<400> 5
cgtatcgcct ccctcgcgcc atcagtgtaa aacgacggcc agt 43

<210> 6
<211> 20
<212> DNA
<213> Artificial

<220>
<223> reverse Complement of SK primer sequence = SK primer sequence

<400> 6
cgctctagaa ctagtggatc 20

<210> 7
<211> 20
<212> DNA
<213> Artificial

<220>
<223> PCR primer

<400> 7
gtggtgagct ctgtaaaacg 20

<210> 8
<211> 21
<212> DNA
<213> Artificial

<220>
<223> PCR primer

<400> 8
cgattcatag atctctgcag g 21

<210> 9
<211> 7292
<212> DNA
<213> Artificial

<220>
<223> vector for inserting barcode sequences

<220>
<221> barcode insertion site
<222> (2619)..(2620)

<400> 9
gcttgcatgc aacttctttt cttttttttt cttttctctc tcccccgttg ttgtctcacc 60

atatccgcaa tgacaaaaaa aatgatggaa gacactaaag gaaaaaatta acgacaaaga 120

cagcaccaac agatgtcgtt gttccagagc tgatgagggg tatcttcgaa cacacgaaac 180

tttttccttc cttcattcac gcacactact ctctaatgag caacggtata cggccttcct 240

tccagttact tgaatttgaa ataaaaaaag tttgccgctt tgctatcaag tataaataga 300

cctgcaatta ttaatctttt gtttcctcgt cattgttctc gttccctttc ttccttgttt 360

ctttttctgc acaatatttc aagctatacc aagcatacaa tcaactccaa gcttgaagca 420

agcctcctga aagatgaagc tactgtcttc tatcgaacaa gcatgcgata tttgccgact 480
taaaaagctc aagtgctcca aagaaaaacc gaagtgcgcc aagtgtctga agaacaactg 540
ggagtgtcgc tactctccca aaaccaaaag gtctccgctg actagggcac atctgacaga 600
agtggaatca aggctagaaa gactggaaca gctatttcta ctgatttttc ctcgagaaga 660
ccttgacatg attttgaaaa tggattcttt acaggatata aaagcattgt taacaggatt 720
atttgtacaa gataatgtga ataaagatgc cgtcacagat agattggctt cagtggagac 780
tgatatgcct ctaacattga gacagcatag aataagtgcg acatcatcat cggaagagag 840
tagtaacaaa ggtcaaagac agttgactgt atcgccggaa ttcccatcaa caagtttgta 900
caaaaaagct gaacgagaaa cgtaaaatga tataaatatc aatatattaa attagatttt 960
gcataaaaaa cagactacat aatactgtaa aacacaacat atccagtcac tatggcggcc 1020
gcattaggca ccccaggctt tacactttat gcttccggct cgtataatgt gtggattttg 1080
agttaggatc cggcgagatt ttcaggagct aaggaagcta aaatggagaa aaaaatcact 1140
ggatatacca ccgttgatat atcccaatgg catcgtaaag aacattttga ggcatttcag 1200
tcagttgctc aatgtaccta taaccagacc gttcagctgg atattacggc ctttttaaag 1260
accgtaaaga aaaataagca caagttttat ccggccttta ttcacattct tgcccgcctg 1320
atgaatgctc atccggaatt ccgtatggca atgaaagacg gtgagctggt gatatgggat 1380
agtgttcacc cttgttacac cgttttccat gagcaaactg aaacgttttc atcgctctgg 1440
agtgaatacc acgacgattt ccggcagttt ctacacatat attcgcaaga tgtggcgtgt 1500
tacggtgaaa acctggccta tttccctaaa gggtttattg agaatatgtt tttcgtctca 1560
gccaatccct gggtgagttt caccagtttt gatttaaacg tggccaatat ggacaacttc 1620
ttcgcccccg ttttcaccat gggcaaatat tatacgcaag gcgacaaggt gctgatgccg 1680
ctggcgattc aggttcatca tgccgtctgt gatggcttcc atgtcggcag aatgcttaat 1740
gaattacaac agtactgcga tgagtggcag ggcggggcgt aaagatctgg atccggctta 1800
ctaaaagcca gataacagta tgcgtatttg cgcgctgatt tttgcggtat aagaatatat 1860
actgatatgt atacccgaag tatgtcaaaa agaggtgtgc tatgaagcag cgtattacag 1920
tgacagttga cagcgacagc tatcagttgc tcaaggcata tatgatgtca atatctccgg 1980
tctggtaagc acaaccatgc agaatgaagc ccgtcgtctg cgtgccgaac gctggaaagc 2040
ggaaaatcag gaagggatgg ctgaggtcgc ccggtttatt gaaatgaacg gctcttttgc 2100
tgacgagaac agggactggt gaaatgcagt ttaaggttta cacctataaa agagagagcc 2160
gttatcgtct gtttgtggat gtacagagtg atattattga cacgcccggg cgacggatgg 2220
tgatccccct ggccagtgca cgtctgctgt cagataaagt ctcccgtgaa ctttacccgg 2280
tggtgcatat cggggatgaa agctggcgca tgatgaccac cgatatggcc agtgtgccgg 2340
tctccgttat cggggaagaa gtggctgatc tcagccaccg cgaaaatgac atcaaaaacg 2400
ccattaacct gatgttctgg ggaatataaa tgtcaggctc ccttatacac agccagtctg 2460

cagggtcgag catagtgact ggatatgttg tgttttacag tattatgtag tctgtttttt 2520
atgcaaaatc taatttaata tattgatatt tatatcattt tacgtttctc gttcagcttt 2580
cttgtacaaa gtggtgagct ctgtaaaacg acggccagtg atccactagt tctagagcgg 2640
cggccgcgcc cgggcaccgg tgtcgacctg cagccaagct aattccgggc gaatttctta 2700
tgatttatga tttttattat taaataagtt ataaaaaaaa taagtgtata caaattttaa 2760
agtgactctt aggttttaaa acgaaaattc ttgttcttga gtaactcttt cctgtaggtc 2820
aggttgcttt ctcaggtata gcatgaggtc gctcttattg accacacctc taccggcatg 2880
ccggcaagtg cacaaacaat acttaaataa atactactca gtaataacct atttcttagc 2940
atttttgacg aaatttgcta ttttgttaga gtcttttaca ccatttgtct ccacacctcc 3000
gcttacatca acaccaataa cgccatttaa tctaagcgca tcaccaacat tttctggcgt 3060
cagtccacca gctaacataa aatgtaagct ttcggggctc tcttgccttc caacccagtc 3120
agaaatcgag ttccaatcca aaagttcacc tgtcccacct gcttctgaat caaacaaggg 3180
aataaacgaa tgaggtttct gtgaagctgc actgagtagt atgttgcagt cttttggaaa 3240
tacgagtctt ttaataactg gcaaaccgag gaactcttgg tattcttgcc acgactcatc 3300
tccatgcagt tggacgatat caatgccgta atcattgacc agagccaaaa catcctcctt 3360
aggttgatta cgaaacacgc caaccaagta tttcggagtg cctgaactat ttttatatgc 3420
ttttacaaga cttgaaattt tccttgcaat aaccgggtca attgttctct ttctattggg 3480
cacacatata atacccagca agtcagcatc ggaatctaga gcacattctg cggcctctgt 3540
gctctgcaag ccgcaaactt tcaccaatgg accagaacta cctgtgaaat taataacaga 3600
catactccaa gctgcctttg tgtgcttaat cacgtatact cacgtgctca atagtcacca 3660
atgccctccc tcttggccct ctccttttct tttttcgacc gaattaattc gtaatcatgg 3720
tcatagctgt ttcctgtgtg aaattgttat ccgctcacaa ttccacacaa catacgagcc 3780
ggaagcataa agtgtaaagc ctggggtgcc taatgagtga ggtaactcac attaattgcg 3840
ttgcgctcac tgcccgcttt ccagtcggga aacctgtcgt gccagctgga ttaatgaatc 3900
ggccaacgcg cggggagagg cggtttgcgt attgggcgct cttccgcttc ctcgctcact 3960
gactcgctgc gctcggtcgt tcggctgcgg cgagcggtat cagctcactc aaaggcggta 4020
atacggttat ccacagaatc aggggataac gcaggaaaga acatgtgagc aaaaggccag 4080
caaaaggcca ggaaccgtaa aaaggccgcg ttgctggcgt ttttccatag gctccgcccc 4140
cctgacgagc atcacaaaaa tcgacgctca agtcagaggt ggcgaaaccc gacaggacta 4200
taaagatacc aggcgtttcc ccctggaagc tccctcgtgc gctctcctgt tccgaccctg 4260
ccgcttaccg gatacctgtc cgcctttctc ccttcgggaa gcgtggcgct ttctcatagc 4320
tcacgctgta ggtatctcag ttcggtgtag gtcgttcgct ccaagctggg ctgtgtgcac 4380
gaaccccccg ttcagcccga ccgctgcgcc ttatccggta actatcgtct tgagtccaac 4440
ccggtaagac acgacttatc gccactggca gcagccactg gtaacaggat tagcagagcg 4500

aggtatgtag gcggtgctac agagttcttg aagtggtggc ctaactacgg ctacactaga 4560
aggacagtat ttggtatctg cgctctgctg aagccagtta ccttcggaaa aagagttggt 4620
agctcttgat ccggcaaaca aaccaccgct ggtagcggtg gtttttttgt ttgcaagcag 4680
cagattacgc gcagaaaaaa aggatctcaa gaagatcctt tgatcttttc tacggggtct 4740
gacgctcagt ggaacgaaaa ctcacgttaa gggattttgg tcatgagatt atcaaaaagg 4800
atcttcacct agatcctttt aaattaaaaa tgaagtttta aatcaatcta aagtatatat 4860
gagtaaactt ggtctgacag ttaccaatgc ttaatcagtg aggcacctat ctcagcgatc 4920
tgtctatttc gttcatccat agttgcctga ctccccgtcg tgtagataac tacgatacgg 4980
gagggcttac catctggccc cagtgctgca atgataccgc gagacccacg ctcaccggct 5040
ccagatttat cagcaataaa ccagccagcc ggaagggccg agcgcagaag tggtcctgca 5100
actttatccg cctccatcca gtctattaat tgttgccggg aagctagagt aagtagttcg 5160
ccagttaata gtttgcgcaa cgttgttgcc attgctacag gcatcgtggt gtcacgctcg 5220
tcgtttggta tggcttcatt cagctccggt tcccaacgat caaggcgagt tacatgatcc 5280
cccatgttgt gcaaaaaagc ggttagctcc ttcggtcctc cgatcgttgt cagaagtaag 5340
ttggccgcag tgttatcact catggttatg gcagcactgc ataattctct tactgtcatg 5400
ccatccgtaa gatgcttttc tgtgactggt gagtactcaa ccaagtcatt ctgagaatag 5460
tgtatgcggc gaccgagttg ctcttgcccg gcgtcaatac gggataatac cgcgccacat 5520
agcagaactt taaaagtgct catcattgga aaacgttctt cggggcgaaa actctcaagg 5580
atcttaccgc tgttgagatc cagttcgatg taacccactc gtgcacccaa ctgatcttca 5640
gcatctttta ctttcaccag cgtttctggg tgagcaaaaa caggaaggca aaatgccgca 5700
aaaaagggaa taagggcgac acggaaatgt tgaatactca tactcttcct ttttcaatat 5760
tattgaagca tttatcaggg ttattgtctc atgagcggat acatatttga atgtatttag 5820
aaaaataaac aaataggggt tccgcgcaca tttccccgaa aagtgccacc tgacgtctaa 5880
gaaaccatta ttatcatgac attaacctat aaaaataggc gtatcacgag gccctttcgt 5940
ctcgcgcgtt tcggtgatga cggtgaaaac ctctgacaca tgcagctccc ggagacggtc 6000
acagcttgtc tgtaagcgga tgccgggagc agacaagccc gtcagggcgc gtcagcgggt 6060
gttggcgggt gtcggggctg gcttaactat gcggcatcag agcagattgt actgagagtg 6120
caccataacg catttaagca taaacacgca ctatgccgtt cttctcatgt atatatatat 6180
acaggcaaca cgcagatata ggtgcgacgt gaacagtgag ctgtatgtgc gcagctcgcg 6240
ttgcattttc ggaagcgctc gttttcggaa acgctttgaa gttcctattc cgaagttcct 6300
attctctagc tagaaagtat aggaacttca gagcgctttt gaaaaccaaa agcgctctga 6360
agacgcactt tcaaaaaacc aaaaacgcac cggactgtaa cgagctacta aaatattgcg 6420
aataccgctt ccacaaacat tgctcaaaag tatctctttg ctatatatct ctgtgctata 6480
tccctatata acctacccat ccacctttcg ctccttgaac ttgcatctaa actcgacctc 6540

tacatttttt atgtttatct ctagtattac tctttagaca aaaaaattgt agtaagaact 6600

attcatagag tgaatcgaaa acaatacgaa aatgtaaaca tttcctatac gtagtatata 6660

gagacaaaat agaagaaacc gttcataatt ttctgaccaa tgaagaatca tcaacgctat 6720

cactttctgt tcacaaagta tgcgcaatcc acatcggtat agaatataat cggggatgcc 6780

tttatcttga aaaaatgcac ccgcagcttc gctagtaatc agtaaacgcg ggaagtggag 6840

tcaggctttt tttatggaag agaaaataga caccaaagta gccttcttct aaccttaacg 6900

gacctacagt gcaaaaagtt atcaagagac tgcattatag agcgcacaaa ggagaaaaaa 6960

agtaatctaa gatgctttgt tagaaaaata gcgctctcgg gatgcatttt tgtagaacaa 7020

aaaagaagta tagattcttt gttggtaaaa tagcgctctc gcgttgcatt tctgttctgt 7080

aaaaatgcag ctcagattct ttgtttgaaa aattagcgct ctcgcgttgc atttttgttt 7140

tacaaaaatg aagcacagat tcttcgttgg taaaatagcg ctttcgcgtt gcatttctgt 7200

tctgtaaaaa tgcagctcag attctttgtt tgaaaattta gcgctctcgc gttgcatttt 7260

tgttctacaa aatgaagcac agatgcttcg tt 7292

<210> 10
<211> 8433
<212> DNA
<213> Artificial

<220>
<223> vector allowing insertion of barcode sequences


<220>
<221> barcode insertion site
<222> (2576)..(2577)

<400> 10
gcttgcatgc aacttctttt cttttttttt cttttctctc tcccccgttg ttgtctcacc 60

atatccgcaa tgacaaaaaa aatgatggaa gacactaaag gaaaaaatta acgacaaaga 120

cagcaccaac agatgtcgtt gttccagagc tgatgagggg tatcttcgaa cacacgaaac 180

tttttccttc cttcattcac gcacactact ctctaatgag caacggtata cggccttcct 240

tccagttact tgaatttgaa ataaaaaaag tttgccgctt tgctatcaag tataaataga 300

cctgcaatta ttaatctttt gtttcctcgt cattgttctc gttccctttc ttccttgttt 360

ctttttctgc acaatatttc aagctatacc aagcatacaa tcaactccaa gctttgcaaa 420

gatggataaa gcggaattaa ttcccgagcc tccaaaaaag aagagaaagg tcgaattggg 480

taccgccgcc aattttaatc aaagtgggaa tattgctgat agctcattgt ccttcacttt 540

cactaacagt agcaacggtc cgaacctcat aacaactcaa acaaattctc aagcgctttc 600

acaaccaatt gcctcctcta acgttcatga taacttcatg aataatgaaa tcacggctag 660

taaaattgat gatggtaata attcaaaacc actgtcacct ggttggacgg accaaactgc 720

gtataacgcg tttggaatca ctacagggat gtttaatacc actacaatgg atgatgtata 780

taactatcta ttcgatgatg aagatacccc accaaaccca aaaaaagaga tcgaattccc 840

atcaacaagt ttgtacaaaa aagctgaacg agaaacgtaa aatgatataa atatcaatat 900
attaaattag attttgcata aaaaacagac tacataatac tgtaaaacac aacatatcca 960
gtcactatgg cggccgcatt aggcacccca ggctttacac tttatgcttc cggctcgtat 1020
aatgtgtgga ttttgagtta ggatccggcg agattttcag gagctaagga agctaaaatg 1080
gagaaaaaaa tcactggata taccaccgtt gatatatccc aatggcatcg taaagaacat 1140
tttgaggcat ttcagtcagt tgctcaatgt acctataacc agaccgttca gctggatatt 1200
acggcctttt taaagaccgt aaagaaaaat aagcacaagt tttatccggc ctttattcac 1260
attcttgccc gcctgatgaa tgctcatccg gaattccgta tggcaatgaa agacggtgag 1320
ctggtgatat gggatagtgt tcacccttgt tacaccgttt tccatgagca aactgaaacg 1380
ttttcatcgc tctggagtga ataccacgac gatttccggc agtttctaca catatattcg 1440
caagatgtgg cgtgttacgg tgaaaacctg gcctatttcc ctaaagggtt tattgagaat 1500
atgtttttcg tctcagccaa tccctgggtg agtttcacca gttttgattt aaacgtggcc 1560
aatatggaca acttcttcgc ccccgttttc accatgggca aatattatac gcaaggcgac 1620
aaggtgctga tgccgctggc gattcaggtt catcatgccg tctgtgatgg cttccatgtc 1680
ggcagaatgc ttaatgaatt acaacagtac tgcgatgagt ggcagggcgg ggcgtaaaga 1740
tctggatccg gcttactaaa agccagataa cagtatgcgt atttgcgcgc tgatttttgc 1800
ggtataagaa tatatactga tatgtatacc cgaagtatgt caaaaagagg tgtgctatga 1860
agcagcgtat tacagtgaca gttgacagcg acagctatca gttgctcaag gcatatatga 1920
tgtcaatatc tccggtctgg taagcacaac catgcagaat gaagcccgtc gtctgcgtgc 1980
cgaacgctgg aaagcggaaa atcaggaagg gatggctgag gtcgcccggt ttattgaaat 2040
gaacggctct tttgctgacg agaacaggga ctggtgaaat gcagtttaag gtttacacct 2100
ataaaagaga gagccgttat cgtctgtttg tggatgtaca gagtgatatt attgacacgc 2160
ccgggcgacg gatggtgatc cccctggcca gtgcacgtct gctgtcagat aaagtctccc 2220
gtgaacttta cccggtggtg catatcgggg atgaaagctg gcgcatgatg accaccgata 2280
tggccagtgt gccggtctcc gttatcgggg aagaagtggc tgatctcagc caccgcgaaa 2340
atgacatcaa aaacgccatt aacctgatgt tctggggaat ataaatgtca ggctccctta 2400
tacacagcca gtctgcaggt cgagcatagt gactggatat gttgtgtttt acagtattat 2460
gtagtctgtt ttttatgcaa aatctaattt aatatattga tatttatatc attttacgtt 2520
tctcgttcag ctttcttgta caaagtggtg agctcgatcc actagttcta gagcgctgag 2580
cgggctggca aggcgcatag gcggccgcgc ccgggcaccg gtgtcgacct gcagagatct 2640
atgaatcgta gatactgaaa aaccccgcaa gttcacttca actgtgcatc gtgcaccatc 2700
tcaatttctt tcatttatac atcgttttgc cttcttttat gtaactatac tcctctaagt 2760
ttcaatcttg gccatgtaac ctctgatcta tagaattttt taaatgacta gaattaatgc 2820
ccatcttttt tttggaccta aattcttcat gaaaatatat tacgagggct tattcagaag 2880

ctttggactt cttcgccaga ggtttggtca agtctccaat caaggttgtc ggcttgtcta 2940
ccttgccaga aatttacgaa aagatggaaa agggtcaaat cgttggtaga tacgttgttg 3000
acacttctaa ataagcgaat ttcttatgat ttatgatttt tattattaaa taagttataa 3060
aaaaaataag tgtatacaaa ttttaaagtg actcttaggt tttaaaacga aaattcttgt 3120
tcttgagtaa ctctttcctg taggtcaggt tgctttctca ggtatagcat gaggtcgctc 3180
ttattgacca cacctctacc ggcatgcccg aaattcccct accctatgaa catattccat 3240
tttgtaattt cgtgtcgttt ctattatgaa tttcatttat aaagtttatg tacaaatatc 3300
ataaaaaaag agaatctttt taagcaagga ttttcttaac ttcttcggcg acagcatcac 3360
cgacttcggt ggtactgttg gaaccaccta aatcaccagt tctgatacct gcatccaaaa 3420
cctttttaac tgcatcttca atggccttac cttcttcagg caagttcaat gacaatttca 3480
acatcattgc agcagacaag atagtggcga tagggtcaac cttattcttt ggcaaatctg 3540
gagcagaacc gtggcatggt tcgtacaaac caaatgcggt gttcttgtct ggcaaagagg 3600
ccaaggacgc agatggcaac aaacccaagg aacctgggat aacggaggct tcatcggaga 3660
tgatatcacc aaacatgttg ctggtgatta taataccatt taggtgggtt gggttcttaa 3720
ctaggatcat ggcggcagaa tcaatcaatt gatgttgaac cttcaatgta ggaaattcgt 3780
tcttgatggt ttcctccaca gtttttctcc ataatcttga agaggccaaa acattagctt 3840
tatccaagga ccaaataggc aatggtggct catgttgtag ggccatgaaa gcggccattc 3900
ttgtgattct ttgcacttct ggaacggtgt attgttcact atcccaagcg acaccatcac 3960
catcgtcttc ctttctctta ccaaagtaaa tacctcccac taattctctg acaacaacga 4020
agtcagtacc tttagcaaat tgtggcttga ttggagataa gtctaaaaga gagtcggatg 4080
caaagttaca tggtcttaag ttggcgtaca attgaagttc tttacggatt tttagtaaac 4140
cttgttcagg tctaacacta cctgtacccc atttaggacc acccacagca cctaacaaaa 4200
cggcatcaac cttcttggag gcttccagcg cctcatctgg aagtgggaca cctgtagcat 4260
cgatagcagc accaccaatt aaatgatttt cgaatcgaac cttgacattg gaacgaacat 4320
cagaaatagc tttaagaacc ttaatggctt cggctgtgat ttcttgacca acgtggtcac 4380
ctggcaaaac gacgatcttc ttaggggcag acattagaat ggtatatcct tgaaatatat 4440
atatatattg ctgaaatgta aaaggtaaga aaagttagaa agtaagacga ttgctaacca 4500
cctattggaa aaaacaatag gtccttaaat aatattgtca acttcaagta ttgtgatgca 4560
agcatttagt catgaacgct tctctattct atatgaaaag ccggttccgg cctctcacct 4620
ttcctttttc tcccaatttt tcagttgaaa aaggtatatg cgtcaggcga cctctgaaat 4680
taacaaaaaa tttccagtca tcgaatttga ttctgtgcga tagcgcccct gtgtgttctc 4740
gttatgttga ggaaaaaaat aatggttgct aagagattcg aactcttgca tcttacgata 4800
cctgagtatt cccacagttg gggatctcga ctctagctag aggatcaatt cgtaatcatg 4860
gtcatagctg tttcctgtgt gaaattgtta tccgctcaca attccacaca acatacgagc 4920

cggaagcata aagtgtaaag cctggggtgc ctaatgagtg aggtaactca cattaattgc 4980
gttgcgctca ctgcccgctt tccagtcggg aaacctgtcg tgccagctgg attaatgaat 5040
cggccaacgc gcggggagag gcggtttgcg tattgggcgc tcttccgctt cctcgctcac 5100
tgactcgctg cgctcggtcg ttcggctgcg gcgagcggta tcagctcact caaaggcggt 5160
aatacggtta tccacagaat caggggataa cgcaggaaag aacatgtgag caaaaggcca 5220
gcaaaaggcc aggaaccgta aaaaggccgc gttgctggcg tttttccata ggctccgccc 5280
ccctgacgag catcacaaaa atcgacgctc aagtcagagg tggcgaaacc cgacaggact 5340
ataaagatac caggcgtttc cccctggaag ctccctcgtg cgctctcctg ttccgaccct 5400
gccgcttacc ggatacctgt ccgcctttct cccttcggga agcgtggcgc tttctcatag 5460
ctcacgctgt aggtatctca gttcggtgta ggtcgttcgc tccaagctgg gctgtgtgca 5520
cgaacccccc gttcagcccg accgctgcgc cttatccggt aactatcgtc ttgagtccaa 5580
cccggtaaga cacgacttat cgccactggc agcagccact ggtaacagga ttagcagagc 5640
gaggtatgta ggcggtgcta cagagttctt gaagtggtgg cctaactacg gctacactag 5700
aaggacagta tttggtatct gcgctctgct gaagccagtt accttcggaa aaagagttgg 5760
tagctcttga tccggcaaac aaaccaccgc tggtagcggt ggtttttttg tttgcaagca 5820
gcagattacg cgcagaaaaa aaggatctca agaagatcct ttgatctttt ctacggggtc 5880
tgacgctcag tggaacgaaa actcacgtta agggattttg gtcatgagat tatcaaaaag 5940
gatcttcacc tagatccttt taaattaaaa atgaagtttt aaatcaatct aaagtatata 6000
tgagtaaact tggtctgaca gttaccaatg cttaatcagt gaggcaccta tctcagcgat 6060
ctgtctattt cgttcatcca tagttgcctg actccccgtc gtgtagataa ctacgatacg 6120
ggagggctta ccatctggcc ccagtgctgc aatgataccg cgagacccac gctcaccggc 6180
tccagattta tcagcaataa accagccagc cggaagggcc gagcgcagaa gtggtcctgc 6240
aactttatcc gcctccatcc agtctattaa ttgttgccgg gaagctagag taagtagttc 6300
gccagttaat agtttgcgca acgttgttgc cattgctaca ggcatcgtgg tgtcacgctc 6360
gtcgtttggt atggcttcat tcagctccgg ttcccaacga tcaaggcgag ttacatgatc 6420
ccccatgttg tgcaaaaaag cggttagctc cttcggtcct ccgatcgttg tcagaagtaa 6480
gttggccgca gtgttatcac tcatggttat ggcagcactg cataattctc ttactgtcat 6540
gccatccgta agatgctttt ctgtgactgg tgagtactca accaagtcat tctgagaata 6600
gtgtatgcgg cgaccgagtt gctcttgccc ggcgtcaata cgggataata ccgcgccaca 6660
tagcagaact ttaaaagtgc tcatcattgg aaaacgttct tcggggcgaa aactctcaag 6720
gatcttaccg ctgttgagat ccagttcgat gtaacccact cgtgcaccca actgatcttc 6780
agcatctttt actttcacca gcgtttctgg gtgagcaaaa acaggaaggc aaaatgccgc 6840
aaaaaaggga ataagggcga cacggaaatg ttgaatactc atactcttcc tttttcaata 6900
ttattgaagc atttatcagg gttattgtct catgagcgga tacatatttg aatgtattta 6960

gaaaaataaa caaatagggg ttccgcgcac atttccccga aaagtgccac ctgacgtcta 7020
agaaaccatt attatcatga cattaaccta taaaaatagg cgtatcacga ggccctttcg 7080
tctcgcgcgt ttcggtgatg acggtgaaaa cctctgacac atgcagctcc cggagacggt 7140
cacagcttgt ctgtaagcgg atgccgggag cagacaagcc cgtcagggcg cgtcagcggg 7200
tgttggcggg tgtcggggct ggcttaacta tgcggcatca gagcagattg tactgagagt 7260
gcaccataac gcatttaagc ataaacacgc actatgccgt tcttctcatg tatatatata 7320
tacaggcaac acgcagatat aggtgcgacg tgaacagtga gctgtatgtg cgcagctcgc 7380
gttgcatttt cggaagcgct cgttttcgga aacgctttga agttcctatt ccgaagttcc 7440
tattctctag ctagaaagta taggaacttc agagcgcttt tgaaaaccaa aagcgctctg 7500
aagacgcact ttcaaaaaac caaaaacgca ccggactgta acgagctact aaaatattgc 7560
gaataccgct tccacaaaca ttgctcaaaa gtatctcttt gctatatatc tctgtgctat 7620
atccctatat aacctaccca tccacctttc gctccttgaa cttgcatcta aactcgacct 7680
ctacattttt tatgtttatc tctagtatta ctctttagac aaaaaaattg tagtaagaac 7740
tattcataga gtgaatcgaa aacaatacga aaatgtaaac atttcctata cgtagtatat 7800
agagacaaaa tagaagaaac cgttcataat tttctgacca atgaagaatc atcaacgcta 7860
tcactttctg ttcacaaagt atgcgcaatc cacatcggta tagaatataa tcggggatgc 7920
ctttatcttg aaaaaatgca cccgcagctt cgctagtaat cagtaaacgc gggaagtgga 7980
gtcaggcttt ttttatggaa gagaaaatag acaccaaagt agccttcttc taaccttaac 8040
ggacctacag tgcaaaaagt tatcaagaga ctgcattata gagcgcacaa aggagaaaaa 8100
aagtaatcta agatgctttg ttagaaaaat agcgctctcg ggatgcattt ttgtagaaca 8160
aaaaagaagt atagattctt tgttggtaaa atagcgctct cgcgttgcat ttctgttctg 8220
taaaaatgca gctcagattc tttgtttgaa aaattagcgc tctcgcgttg catttttgtt 8280
ttacaaaaat gaagcacaga ttcttcgttg gtaaaatagc gctttcgcgt tgcatttctg 8340
ttctgtaaaa atgcagctca gattctttgt ttgaaaaatt agcgctctcg cgttgcattt 8400
ttgttctaca aaatgaagca cagatgcttc gtt 8433